# EUROPEAN PATENT SPECIFICATION

(11) **EP 3 866 615 B1**
(45) Date of publication and mention of the grant of the patent: **30.08.2023**
(21) Application number: 19874358.5
(22) Date of filing: 14.10.2019
(51) Int. Cl.: A61K 31/198, A61K 31/716, A61K 31/702, A61K 31/718, A61K 31/685, A23L 33/10, A23L 33/175, A23L 33/12, A23L 33/125, A23L 33/15, A23L 33/16, A61P 29/00, A61P 1/00, A61P 25/22, A61P 25/28

(54) **DIETARY SUPPLEMENT FOR TREATING DYSBIOSIS**
NAHRUNGSERGÄNZUNG ZUR BEHANDLUNG VON DYSBIOSE
COMPLÉMENT ALIMENTAIRE POUR LE TRAITEMENT DE LA DYSBIOSE

(30) Priority: 15.10.2018 US 201816160658
(43) Date of publication of application: 25.08.2021
(73) Proprietor: Freedom Health, LLC, Aurora OH 44202 (US)
(72) Inventor: ANDERSON, Scott, Hudson, Ohio 44236 (US); HALL, John, Hudson, Ohio 44236-2295 (US); YOHO, Mark, Chagrin Falls, Ohio 44023 (US)
(74) Representative: Barker Brettell LLP
(86) International application number: PCT/US2019/056045
(87) International publication number: WO 2020/081417

(56) References cited:
- WO-A1-2014/085684
- WO-A1-2018/158405
- US-A1- 2005 058 671
- US-A1- 2005 058 671
- US-A1- 2010 284 972
- US-A1- 2018 015 032
- Anonymous: "GastroEase EQ(TM) Total Digestive Support Powder | Perfect Products EQ", , 11 November 2015 (2015-11-11), XP55917270, Retrieved from the Internet: URL:https://perfectproductseq.com/gastroea se-eq-powder/#product-reviews [retrieved on 2022-05-02]
- PEDERSEN M.B. ET AL: "Compositional profile and variation of Distillers Dried Grains with Solubles from various origins with focus on non-starch polysaccharides", ANIMAL FEED SCIENCE AND TECHNOLOGY, vol. 197, 7 November 2014 (2014-11-07), pages 130-141, XP55917271, AMSTERDAM, NL ISSN: 0377-8401, DOI: 10.1016/j.anifeedsci.2014.07.011
- MUDGIL DEEPAK ET AL: "Composition, properties and health benefits of indigestible carbohydrate polymers as dietary fiber: A review", INTERNATIONAL JOURNAL OF BIOLOGICAL MACROMOLECULES, ELSEVIER BV, NL, vol. 61, 2 July 2013 (2013-07-02), pages 1-6, XP028729247, ISSN: 0141-8130, DOI: 10.1016/J.IJBIOMAC.2013.06.044

## Description

### FIELD OF THE INVENTION

This invention generally relates to dietary supplements for humans and animals, and more particularly to a novel dietary supplement for use in treating and/or preventing dysbiosis and diseases associated with dysbiosis.

### BACKGROUND OF THE INVENTION

Researchers are increasingly finding that human gut microbes (collectively known as the microbiota), through their protective effect on the gut lining and their moderating influence on the immune system are major players in maintaining good health. US 2005/0058671 relates to a dietary supplement for the treatment and prevention of digestive system and digestive system-related disorders, said digestive supplement comprising: polar lipid supplement which contains significant amounts of polar lipids and antioxidants; soluble beta-glucan fiber that provides at least one beneficial effect on health; and at least one amino acid that provides at least one beneficial effect on a digestive system.

Humans acquire a microbiota at birth as they come down the birth canal. If they are born via C-section, they instead pick up a microbiota from the nurses and the hospital. Babies that are breastfed are also seeded with bacteria that live in mother's milk, along with prebiotics (oligosaccharides that feed the microbiota). Babies born by C-section that are formula-fed thus miss two important mechanisms to initiate a healthy microbiota.

Illnesses such as food poisoning can also disrupt the gut microbiota. This disruption is called dysbiosis, and it can lead to a thinning of the mucus layer that lines the entire gut as well as damage to the enterocytes that form the gut lining. When the gut lining is breached, bacteria can escape the gut and lead to systemic infection. The body responds by launching an immune attack on the circulating bacteria that can reach every organ in the body. The immune system often produces collateral damage, killing human cells along with the bacteria. If the infection lingers, the inflammation can become chronic.

Long term systemic inflammation has been linked to virtually all chronic diseases, from heart disease to neurological problems. To redress this issue, the gut lining must be healed in order to stop the flow of pathogens into the bloodstream. Two therapies are used to treat dysbiosis: *probiotics* (live beneficial bacteria) and *prebiotics* in the form of various oligosaccharides that nourish beneficial bacteria.

There are six outcomes of gut dysbiosis that we consider here: gut inflammation, systemic inflammation, neurological inflammation, antibiotic-induced inflammation, auto-immunity, and chemotherapy.

### GUT INFLAMMATION

Gut inflammation is the first and most direct effect of dysbiosis. It takes the form of gastric ulcers, irritable bowel syndrome (IBS), inflammatory bowel disease (IBD, consisting of ulcerative colitis and Crohn's disease), colon cancer, and rectal cancer. Each of these syndromes is associated with a leaky gut. Gastric ulcers can currently be treated with proton-pump inhibitors and antacids. However much recent research indicates that both of these treatments, by raising gastric pH, can create a deleterious environment for the colon. There are currently very few options for IBS or IBD, although fecal microbial transplants are starting to be used with varying effect. Cancers of the gut are treated with surgery, chemo, and radiation therapy, which incur their own troubles (see below).

### SYSTEMIC INFLAMMATION

Systemic inflammation gives rise to heart disease, type 2 diabetes, various cancers, allergies, and other organ diseases. Heart disease can be treated with surgery, implanted pacemakers/cardioverters and a variety of drugs, including statins, blood thinners, ACE Inhibitors, and beta blockers. Type 2 diabetes can be controlled with insulin, diet and exercise. Cancers are treated with surgery, chemotherapy and radiation treatments. Allergies can be treated with antihistamines.

### NEUROLOGICAL INFLAMMATION

Neurological inflammation takes longer to evolve from the gut, via various gut-brain pathways. Parkinson's starts with Lewy bodies that infect gut cells and cause constipation before they make their way to the brain over a ten-year span. Alzheimer's may also represent a movement of misfolded amyloid proteins originating in the gut that similarly travel to the brain over time. Depression and anxiety are also linked to dysbiotic guts. There are no cures for Parkinson's or Alzheimer's. Certain symptoms of Parkinson's can be treated with L-dopa, but that can also create dyskinesia. Depression and anxiety can be treated with serotonin reuptake inhibitors (SRIs) and other drugs that attempt to raise brain levels of dopamine, serotonin, gamma-amino butyric acid (GABA), and norepinephrine. These drugs don't work for many people, and when they do, they tend to cause weight gain.

### ANTIBIOTIC-INDUCED INFLAMMATION

Antibiotics have saved millions of lives that would otherwise have been lost to bacterial infection. But today, antibiotics are vastly overused and oral administration (as opposed to parenteral administration) has been shown to, in many hospital settings, actually increase inflammation. In particular, C. diff infections as a consequence of antibiotic administration cause half a million infections a year, and the death rate is close to 30,000 people per year. Oral antibiotics, by virtue of decimating normal gut microbial populations, have been shown to increase gut permeability, allowing live bacteria to enter the bloodstream and create systemic inflammation.

### AUTO-IMMUNITY

Auto-immunity is thought to be a problem caused by mimicry, where bacteria or their products have close similarities to existing body tissues. When the immune system attacks these foreign particles, it can also attack normal tissue that shares certain antigenic properties. These diseases include arthritis, lupus, type 1 diabetes, and multiple sclerosis. There are no cures for these diseases. Type 1 diabetes can be treated with insulin, but drugs for auto-immune diseases are mostly experimental.

### CHEMOTHERAPY AND RADIATION TREATMENTS

Chemotherapy and radiation treatments work by attacking cells that are rapidly dividing, because cancer cells exhibit unusually fast turnover. However, these drugs and treatments are not perfectly targeted, and also kill cells that normally have short lifespans, including hair follicles and the lining of the gut, which renews itself weekly. These cancer treatments, then, can lead to leaky gut and invite the entire array of diseases listed above. Specific diseases directly attributable to cancer treatments include mucositis, stomatitis, cachexia and diarrhea. In the context of cancer therapy, these diseases are refractory to treatment, and the general prognosis is dependent on the length and intensity of the cancer therapy. New cancer treatments that involve immune checkpoint inhibitors depend on a well-balanced microbiota. In this way, proper prebiotic fibers can augment these novel immune-cell therapies.

### LEAKY GUT TISSUES LEADING TO SYSTEM INFLAMMATION

A large percentage of chronic diseases, including heart disease, diabetes, obesity, IBD, IBS, arthritis, Alzheimer's and Parkinson's, are strongly associated with gut dysbiosis, in particular, increased gut permeability - so called "leaky gut". Bacteria that enter the bloodstream can be pumped to each and every organ of the body, thus spreading inflammation system-wide. Inflammation is a putative precursor to the above-mentioned diseases. It follows, then, that a product that can heal a leaky gut can reduce systemic inflammation and lower the incidence of chronic disease.

Prebiotics are known to increase the numbers of beneficial microbes that produce short-chain fatty acids (SCFAs) that in turn can speed the repair and regeneration of damaged tissue in the gut. However, boosting microbes in the presence of a strongly permeable gut lining can risk systemic inflammation.

Because these diseases are all associated with inflammation, success has been achieved by treating them with prebiotics and probiotics. The preponderance of evidence points to the need to balance the microbiota in order to prevent, ameliorate or cure these many chronic diseases of inflammation.

As such, there is a need in the art for a dietary supplement which synergistically addresses the above issues. The invention provides such a dietary supplement. These and other advantages of the invention, as well as additional inventive features, will be apparent from the description of the invention provided herein.

### BRIEF SUMMARY OF THE INVENTION

The references to methods of treatment by therapy of this description are to be interpreted as references to compositions of the present invention for use in those methods.

The primary objective of the present invention is to treat dysbiosis and its attendant effect on the gut lining and ultimately the corresponding immune response in humans and potentially other animals as well. In addition to treating these maladies, the present invention aims to prevent dysbiosis and its attendant illnesses as well.

An additional objective of the present invention is to use only safe ingredients. The present invention is gluten-free, allergen-free, non-GMO, milk-free, egg-free, nut-free, and preservative-free, with no artificial additives. Furthermore, the present invention is intended to be conveniently consumed orally in the form of a paste, a solid (such as a bar, a pressed pill or a biscuit), a liquid or a powder that can be consumed alone or mixed with other foods or drinks. A further objective of the present invention is to make it easy and convenient to ship and store.

The present invention has been designed to be stable with a commercially acceptable shelf life. The present invention is relatively inexpensive compared with drugs that purport to heal the gut, such as proton pump inhibitors. All of the above objectives have been designed to avoid any substantial relative disadvantage to current treatments.

With this invention, a novel dietary supplement that is formulated to treat and/or prevent a number of digestive tract disorders and a number of immune-related disorders as well is provided. Through the periodic administration of this dietary supplement to humans or other animals in accordance with methods taught by the present invention, a number of digestive tract disorders and a number of immune-related disorders are effectively treated and/or prevented in such humans and other animals. As will become apparent to those skilled in the art, the dietary supplement of the present invention is much more than the sum of its ingredients, with the combination of ingredients yielding a synergistic result substantially more efficacious than the results which would be produced if each of the ingredients acting by itself were provided to humans or other animals.

The dietary supplement of the present invention can be manufactured in several different forms, which may either be taken directly as a dietary supplement or added to food or drink. The dietary supplement of the present invention may be manufactured as a solid, granulated solid, powder, paste, or liquid. In order to manufacture it as a solid, a small amount of oat bran or oat flour (or substitutes therefor) are added to thicken it to food bar form or to allow it to be pressed into pill form. By adding a higher percentage of oat bran or oat flour while stirring the mixture, a granular form of the supplement may be manufactured. By adding still more flour, a powder form of the supplement may be manufactured. By adding more oil (oat oil, sunflower oil, safflower oil, or another oil), the mixture can be brought to a paste having the consistency of peanut butter. By adding still more oil, it can be made into a viscous liquid.

The dietary supplement of the present invention may also be manufactured as a liquid, powder or paste and stored in a gelatin capsule (as gelcaps), which makes for a consistent dosage of the dietary supplement. It is desirable that the dietary supplement of the present invention is taken on a regular basis, which in the preferred embodiment is daily or multiple times daily (for example, with meals) in order to maintain an optimal level of the ingredients in the digestive tract.

Upon disclosure of the dietary supplement of the present invention to those skilled in the art, they will immediately appreciate that the dietary supplement is much more than merely the sum of its ingredients.

Depending upon the particular desired application of the dietary supplement of the present invention, additional constituents such as vitamins and minerals may also be added thereto.

It may therefore be seen that the present invention teaches a dietary supplement which efficaciously treats digestive tract disorders as well as immune-related disorders in humans and in other animals as well. The dietary supplement of the present invention consists entirely of safe ingredients rather than drugs. The dietary supplement of the present invention is orally administrable, thereby making its dispensation a simple matter. The dietary supplement of the present invention may be compounded either in a paste form, in a solid form, a liquid form, or in a powdered or granular form which may be added to liquids for delivery. The dietary supplement of the present invention can also be packaged in a manner which makes it both easy to ship, store, and consume.

The dietary supplement of the present invention is stable and has a long shelf life, and requires no special care to be provided by the user throughout its shelf life prior to usage. The dietary supplement of the present invention is also inexpensive relative to previously known digestive tract disorder treatments and immune-related disorder treatments, thereby enhancing its market appeal and affording it the broadest possible market. Finally, all of the aforesaid advantages and objectives of the dietary supplement of the present invention and its method of administration are achieved without incurring any substantial relative disadvantage.

In view of the foregoing, one aspect of the invention presents a dietary supplement for the treatment and prevention of dysbiosis and diseases associated with dysbiosis. The dietary supplement includes:
L-glutamine;
at least one of the following mucogenic amino acids: L-Threonine, L-Serine, L-Proline, L-Cysteine;
Lecithin;
Fructo-oligosaccharide;
Beta-glucan;
RS-4 starch; and
Arabinoxylan oligosaccharide.

In an embodiment according to this aspect, L-glutamine is produced by vegan bacterial fermentation of sugar beets, isolated, purified, and micronized for better and faster absorption. The included L-glutamine comprises between approximately one percent and twenty percent of said dietary supplement by weight.

In an embodiment according to this aspect, the at least one mucogenic amino acid is produced by vegan bacterial fermentation, isolated and purified. Each of the at least one mucogenic amino acid comprises between approximately zero percent and ten percent of said dietary supplement by weight.

In an embodiment according to this aspect, the lecithin is derived from soy oil, oat oil, sunflower oil, safflower oil, or corn oil. The lecithin comprises between approximately one percent and approximately fifteen percent of said dietary supplement by weight.

In an embodiment according to this aspect, the fructo-oligosaccharides are derived from yacon root, chicory root, Jerusalem artichoke, or blue agave. The fructo-oligosaccharides comprise between approximately one percent and approximately forty percent of said dietary supplement by weight.

In an embodiment according to this aspect, the beta-glucan is derived from oats, barley, mushrooms, seaweed, algae, or yeast cell walls. The beta-glucan comprises between approximately one percent and approximately forty percent of said dietary supplement by weight.

In an embodiment according to this aspect, the arabinoxylan oligosaccharide is derived from the bran tissues of wheat, oats, barley, rice, millet, psyllium, flax, or rye. The arabinoxylan oligosaccharide comprises between approximately one percent and approximately forty percent of said dietary supplement by weight.

In an embodiment according to this aspect, the RS-4 starch is derived from oats, yacon root, chicory root, flax, acacia, corn or bacterial fermentation and then subjected to chemical cross-linking in order to decrease digestibility by human acids and enzymes. The RS-4 starch comprises between approximately one percent and approximately forty percent of said dietary supplement by weight.

In an embodiment according to this aspect, the dietary supplement also includes a nutricine that binds to and eliminates pathogenic bacteria in the digestive tract, the nutricine including at least one of pure mannan or mannan oligosaccharide (MOS). The nutricine that binds to and eliminates pathogenic bacteria in the digestive tract comprises between approximately one-half percent and approximately forty percent of said dietary supplement by weight.

In an embodiment according to this aspect, the dietary supplement also includes a medication that is carried with the other ingredients of said dietary supplement, wherein at least one of the absorption or the therapeutic value of said medication is maximized by being taken in conjunction with said dietary supplement.

In embodiments according to this aspect, the dietary supplement may be compounded as solid food bars, as a paste, as a granulated solid, as a powder, as a liquid, and/or as liquid-filled softgel capsules.

In an embodiment according to this aspect, the digestive system disorders are selected from a group comprising ulcers, colitis, irritable bowel syndrome, diverticulosis, diverticulitis, Crohn's disease, mucositis, and stomatitis. In embodiments according to this aspect, wherein said digestive system related disorders are selected from a group consisting of cachexia, lactose intolerance, and dietary insufficiencies in the elderly.

In embodiments according to this aspect, the immune system related disorders are selected from a group comprising arthritis, diabetes, depression, anxiety, and heart disease. In embodiments according to this aspect, the related disorders are selected from a group comprising Alzheimer's, Parkinson's, multiple sclerosis, and other neurodegenerative diseases.

In embodiments according to this aspect, the dietary supplement also includes at least one vitamin from the group consisting of vitamin B6, vitamin B12, Biotin, vitamin C, vitamin D, vitamin E, and Niacin.

In embodiments according to this aspect, the dietary supplement also includes at least one mineral micronutritional additive selected from the group consisting of calcium, chromium, copper, manganese, magnesium, manganese, phosphorus, potassium, selenium, vanadium, and zinc.

In embodiments according to this aspect, taking a daily dosage of between approximately ten grams and approximately sixty grams of said dietary supplement daily provides effective amounts of said lecithin, said beta glucan, and said amino acids.

In another aspect, the invention provides a method for administering the dietary supplement as described above for use in treating and preventing digestive system and immune-related disorders. An embodiment of such a method includes preparing an appropriately sized dose of dietary supplement and administering said dietary supplement on a regular basis. The method may also include repeating said administering step at least once daily.

The disadvantages, limitations and high cost of the existing state of the art discussed above are overcome by the present invention. Other aspects, objectives and advantages of the invention will become more apparent from the following detailed description when taken in conjunction with the accompanying drawings.

### BRIEF DESCRIPTION OF THE DRAWINGS

The accompanying drawings incorporated in and forming a part of the specification illustrate several aspects of the present invention and, together with the description, serve to explain the principles of the invention. In the drawings:

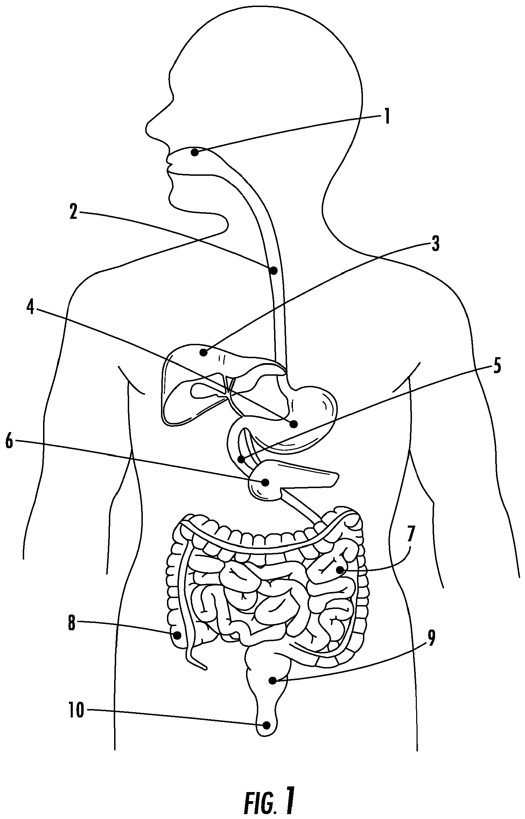

FIG. 1 is a schematic drawing of a human being showing the anatomy of the human digestive tract;
FIG. 2 is a schematic chart showing the prebiotics of the instant invention vs. the portions of the digestive tract and flora which they target; and
FIG. 3 is a plot of amino acid absorption rate against.

### DETAILED DESCRIPTION OF THE INVENTION

Prior to a discussion of the nutritional product of the present invention and methods of making and administering it, it is helpful to briefly discuss the anatomy of the human digestive system. Referring to the Figure, the head and torso of a human being are illustrated together with a schematic illustration of the human digestive tract. The digestive tract of a human being begins at the mouth **1,** and sequentially extends through an esophagus **2** and into a stomach **4.** In the mouth, food is chewed and saliva is mixed with the food to begin digestion of carbohydrates. The food is swallowed, and passes through the esophagus to the stomach, where pepsin assists in the digestion of protein.

From the stomach, the food flows through the duodenum **5,** which is the first portion of the small intestine **7,** where chemicals secreted by the liver **3** and the pancreas **6** enable the duodenum to break down fat. From the duodenum, the food then moves into the small intestine **7,** where the digestion process is completed and intestinal bacteria are found. The digested food then moves to the colon **8,** where water and sodium are removed and where the bulk of the gut microbiota are found, and then to the rectum **9.** The remaining undigested solids then pass from the body through the anus **10.**

The dietary supplement of the present invention includes several principal components, each of which provides a beneficial effect on health which is facilitated by the inclusion of a particular ingredient or a mixture of ingredients in the dietary supplement. During the following discussion of the ingredients of the dietary supplement of the present invention, it will rapidly become apparent to those skilled in the art that the benefits achieved by the dietary supplement of the present invention are substantially greater than the sum of the individual benefits of each of the dietary supplement's ingredients. Briefly, these several components include:
L-glutamine;
One or more polar mucogenic amino acids that increase the number and productivity of goblet cells lining the gut to enhance protection against pathogens in the gut lumen, in particular, at least one of L-Threonine, L-Serine, L-Proline, L-Cysteine;
Lecithin which is included to increase the bioavailability and the rate of absorption of the foregoing polar amino acids so as to allow them to act before the following prebiotics act;
Fructo-oligosaccharide (FOS), a prebiotic operating chiefly in the distal ileum and ascending colon to boost beneficial microbes, especially *Lactobacillus* and *Clostridial* species, in this area of the GI tract;
Beta-glucan, a prebiotic operating chiefly in the ascending and transverse colon, to boost beneficial microbes, especially *Bifidobacteria* and *Faecalibacterial* species, in this area of the GI tract;
RS-4, a chemically modified resistant maize starch, a prebiotic operating chiefly in the transverse and descending colon, to boost beneficial microbes, especially *Lachnospiraceae* and *Roseburial* species, in this area of the GI tract; and
Arabinoxylan oligosaccharide (AXOS), a prebiotic operating chiefly in the descending colon and rectum, to boost beneficial microbes, especially *Veilonella* and *Prevotella* species, in this area of the GI tract.

As will be understood from the disclosure herein, the invention presents effectively a two-part formula. This first part of this formula includes L-glutamine, one or more mucogenic amino acids, and lecithin. These constituents first act to heal the gut by enhancing tight junctions and improving the mucus layer in the gut. An enhanced absorption rate of the amino acids is provided via the inclusion of lecithin. The second part of this formula includes the prebiotics, which improve the gut microbiota as discussed herein. There is a synergistic effect between these two parts. Without first healing the gut, tightening cell junctions, and enhancing the mucosa, there is a risk that the prebiotics may escape the gut and thus not provide their beneficial effects.

Glutamine and the one or more mucogenic amino acids work in concert with one another to aid in nutrient absorption. In particular, people with permeable guts may not benefit from the inclusion of the aforementioned prebiotics. The extra induced bacteria may leak into the bloodstream through the gut lining. The addition of L-glutamine and a mucogenic amino acid helps to quickly heal the gut lining so that extra commensal bacteria will stay in the gut. Further, the Applicant has found that the mucogenic amino acid increases mucosa to thus improve the fidelity of the gut lining. This in turn reduces or eliminates the likelihood that the extra commensal bacteria will escape the gut. L-glutamine used herein may, for non-limiting example, be produced by vegan bacterial fermentation of sugar beets, isolated, purified, and micronized.

L-glutamine is a preferred food of the enterocytes and colonocytes lining the gut. It both nourishes and heals the gut lining, preventing permeability of these tissues by increasing the number of tight-junction proteins that bind colonocytes together, preventing pathogenic organisms from entering the circulatory system. L-glutamine is considered to be a conditionally essential amino acid under normal conditions, because the body can create as much as is needed without the intake of glutamine supplements. But when the digestive system is stressed-for instance by ulcers-large amounts of L-glutamine are consumed, and supplements may be needed to replenish the supply.

L-glutamine is a naturally produced nonessential amino acid which is produced by breaking down protein. L-glutamine is the most abundant amino acid in the bloodstream, and is primarily formed and stored in skeletal muscle and the lungs (and is the primary fuel of enterocytes, essential in their growth, reproduction, and repair). L-glutamine also increases growth hormones, and when ingested has a substantial effect on maintaining and increasing mucosal integrity, including enhancing the integrity of the mucous gut membrane. L-glutamine functions to "kick start" the formation of nucleotides, which are involved in the production of cell tissue and the maturation of the intestinal mucosa, and are directly involved in the immune processes and the energy systems. A diet deficient in glutamine will most likely also likely result in a deficiency in nucleotide formation. Thus, L-threonine and L-glutamine both act to protect the inside wall of the stomach by enhancing the integrity of the mucous gut membrane.

While any one of the listed mucogenic amino acids listed above are suitable, the Applicant has found that L-Threonine works particularly well. L-Threonine is a naturally produced essential amino acid and is an important component of the chemical pathway that creates mucin produced by the goblet cells distributed throughout the intestinal tract. Other amino acids that contribute to the formation of mucin include serine, leucine, isoleucine, and cysteine. By assisting metabolism and nutrient absorption, threonine contributes to a smoothly functioning digestive tract. A deficiency of threonine slows the regeneration of the gut wall and depresses the production of mucus. L-Threonine is especially useful for wound healing and for treating stress, but it is also an essential link in the production of immunoglobulins, enhancing immune function.

L-threonine and L-glutamine, which are both naturally produced amino acids which are produced by breaking down protein, provide additional advantages as well. L-threonine makes up collagen, elastin, and enamel protein, assists in metabolism and assimilation, and aids the digestive system by increasing the integrity of the mucous gut membrane. L-threonine has also been observed by the inventors to have a synergistic effect with beta-glucan in further slowing motility through the stomach. L-threonine and L-glutamine are widely available from a large number of different suppliers, and are also powders.

Lecithin acts as an emulsifier which increases the bioavailability of polar and fat-soluble nutrients (including glutamine, threonine, serine and cysteine as well as many drugs) into the enterocytes lining the gut. This polar lipid protects and strengthens the intestinal tissue of the digestive system and augments the protective effect of mucus in the digestive tract. This Lecithin may be derived from vegetable oil, including the oils of safflower, corn, sunflower, oat or soy. Alternatively, it may be derived from alcohol-extraction oat oil. Optionally, different vegetable oils such as sunflower oil, safflower oil, olive oil, corn oil, or soy oil may be blended in order to vary the amount of polar lipids contained in the polar lipid. A form of the dietary supplement which is to be compounded in a liquid form may contain a mixture of lecithin and sunflower oil (since the sunflower oil does not contain a high amount of polar lipids, it may be thought of as an inactive ingredient). Sunflower or other vegetable oil will generally not be included in the dietary supplement if it is to be compounded into a granular or solid form. Among other benefits, polar lipids increase macrophage activity, modulating immune function.

The Applicant has found that the inclusion of lecithin (a polar lipid) serves an important role as it increases the bioavailability of other constituent polar amino acids of the supplement. Polar lipids increase bioavailability via two mechanisms. First, polar lipid digestion products, along with bile salts, may alter the intrinsic transcellular permeability of the colonocytes lining the gut. Second, polar molecules may solubilize with the polar lipid, facilitating movement through the aqueous diffusion layer. The Applicant theorizes that this mechanism is related to the capacity of the polar lipid to attract target polar molecules via the polar residue and then use the lipophilic residue to transport these molecules across the cellular membrane and into the enterocytes and colonocytes lining the gut. The Applicant has found both lecithin and oat oil are superior for this enhanced membrane transport.

Polar lipids, in particular lecithin, thus provide a versatile delivery vehicle for drugs and nutrients. Studies have shown that polar lipids can increase the bioavailability of co-dissolved lipophilic or polar drugs, including steroids, antibiotics, antihistamines and anti-nausea drugs. In addition to their use as emulsifiers, polar lipids physically augment the protective effect of mucus in the digestive tract. There are a number of potential sources of polar lipids that may be used as the polar lipid in the dietary supplement of the present invention. In the preferred embodiment, lecithin from soy oil is used.

Other oils that are also good sources of polar lipids are oat oil, sunflower oil, safflower oil, soybean oil, olive oil, palm oil, corn oil, rapeseed oil, linseed oil, etc. The preferred concentration of polar lipids used in the dietary supplement of the present invention ranges from approximately 1% to approximately 15% of the dietary supplement by weight. A typical value is 10%. The term "approximately" is used throughout this application to allow for typical formulation manufacturing tolerances readily appreciated by those of skill in the art. Unless otherwise specified herein, all percentages of constituents of the dietary supplement are percentages of the dietary supplement by weight.

The Applicant has found that lecithin functions particularly well in increasing the bioavailability rate of absorption of L-glutamine and the (largely polar) mucogenic amino acid(s). Particularly, the Applicant has found that, by including lecithin, the bioavailability and rate of absorption of L-glutamine and the mucogenic amino acid(s) are considerably greater than what they present in nature. As a result, the dietary supplement, particularly its inclusion of lecithin, results in a compound having markedly different characteristics than any of its naturally occurring constituents, or any related naturally occurring combination of constituents.

As mentioned above, embodiments of the present invention also include a number of prebiotics which are provided to target specified microbiota and portions of the GI tract. The inclusion of the prebiotic beta-glucan has several advantages. For example it is a potent stimulator of the immune system. Beta-glucan also lowers LDL cholesterol in the bloodstream. It also binds to other sugars and releases them over a period of time, reducing sugar highs and lows thereby stabilizing blood sugar levels. In the preferred embodiment, the beta-glucan used is the soluble fiber in oats, an oligosaccharide that is found in the kernel of oats and is a powder when dried. Alternative sources of the beta-glucan are barley, yeast, and other vegetable sources. Beta-glucan is a jelling agent that works with gastric juices or water. In an embodiment, the soluble fiber used is beta-glucan that is derived from oats. Other soluble fibers that are also good sources of beta-glucan are those derived from barley or soybeans. Beta-glucan is widely available from a large number of different suppliers, and may be milled as a flour.

The aforementioned blend of prebiotics also includes FOS. The Applicant has also found that the inclusion of FOS also provides a significant advantage. Indeed, butyrate is known as an important nutrient for enterocytes and colonocytes. In fact butyrate is known to be an anti-carcinogen and the preferred food for the gut lining, helping to heal cells and strengthen the tight junctions between them, limiting permeability. However, butyrate has the odor of rancid butter and is unpalatable. It is therefore not desirable to introduce butyrate as a direct constituent of the supplement. The inclusion of FOS promotes the growth of certain commensal bacteria that in turn produce butyrate. In other words, the inclusion of FOS allows for the generation of butyrate internally and its attendant benefits are achieved. Thus, another advantage of the invention is the introduction of butyrate without the unpalatable odor of the same.

The aforementioned blend of prebiotics also includes RS-4 and Arabinoxylan oligosaccharide. RS-4, a chemically modified resistant maize starch, is a prebiotic operating chiefly in the transverse and descending colon, to boost beneficial microbes, especially *Lachnospiraceae* and *Roseburial* species, in this area of the GI tract. Arabinoxylan oligosaccharide (AXOS), is a prebiotic operating chiefly in the descending colon and rectum, to boost beneficial microbes, especially *Veilonella* and *Prevotella* species, in this area of the GI tract.

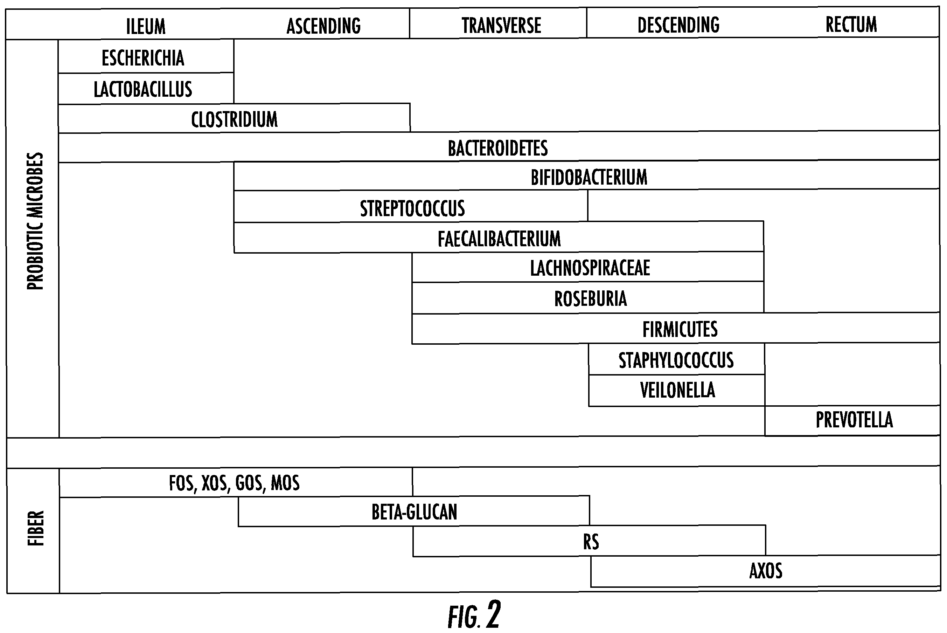

As a result, a spectrum of prebiotics are provided in the dietary supplement, each of which acts in a defined range of pH and oxygenation to target microbes located in distinct segments of the GI tract, from the distal ileum to the rectum. Indeed, FOS operates primarily in the distal ileum and ascending colon. Beta-glucan operates primarily in the ascending and transverse colon. RS-4 operates primarily in the transverse and descending colon. AXOS operates primarily in the descending colon and the rectum. As such, the dietary supplement according to the instant invention targets specific microbes that are found in different areas of the GI tract to provide overlapping coverage, segment to segment. This spectrum is summarized in FIG. 2.

Prebiotics are complex sugars that are not digested by the human enzymes of the GI tract. These sugars thus enter the colon intact and represent an energy source for the colonic microbiota, including in particular certain *Lactobacillus* and *Bifidobacterium* species. These microbes act as keystone species maintaining a balanced gut homeostasis through the formation of short-chain fatty acids. They lay the foundation for cross-feeding interactions with other commensals, including beneficial species of *Clostridium*, *Ruminococcus*, and *Eubacterium.*

The fermentation of prebiotics lowers the pH in the gut, inhibiting peptide degradation and the consequent production of toxic compounds including ammonia and amines and decreases the activity of dysbiotic bacterial enzymes. Some types of commonly compounded prebiotics, including inulin, produce excess gas and bloating, and are therefore excluded in the present invention.

The Applicant has conducted necropsies on 111 horses, collecting bacterial swabs from different areas of the colon. The Applicant found that as the colonic environment changed from pH 5.5 to pH 7 at the distal end, the types of bacteria that populated those various quadrants of the colon changed. The Applicant also found that oxygen was depleted with distance through the gut, favoring anaerobes toward the distal end. The Applicant has found a similar distribution of microbes in the human gut, again tracking with higher pH and lower oxygen toward the distal end.

Each type of prebiotic in the present invention targets specific microbes occupying these unique pH and anaerobic habitats. Each of these niches exhibits distinct levels of acidity and oxygenation. A single prebiotic is not capable of targeting these highly varied environments, but the arbitrary mix of prebiotics found in many products is also not ideal. Instead the present invention targets five specific habitats, each with a distinct pH and oxygen range, roughly corresponding to the ileum, ascending, transverse and descending colon and the rectum. In addition, the prebiotics selected primarily affect keystone species that have a larger impact on microbial communities. This novel approach allows for the treatment of permeability issues throughout the entire colon, as well as the distal ileum.

Oligosaccharides (OS) such as XOS, GOS and FOS increases numbers of *Bifidobacterium* (bifidogenic) and *Lactobacillus* species while lowering numbers of pathogenic *E coli*, *enterococci*, *Clostridium difficile*, and *Clostridium perfringens.* FOS increases numbers of butyrate-producing species, including *F. prausnitzii*, *E. rectale* and *R. inulinovorans.* FOS works best for a bifidogenic effect at pH 6.8, while GOS works best for a bifidogenic effect at pH 6.

Arabinoxylan oligosaccharides (AXOS) derived from oat bran act on Bifidobacterium species in the distal colon and stimulate propionate-producing microbes. Although *Bifidobacterium* species do not produce butyrate, they produce acetate and lactate that are metabolized by *Anaerostipes*, *Eubacterium hallii* and other species that produce butyrate.

These oligosaccharides have an impact on the entire colon, but primarily affect the distal ileum and the ascending colon, with the exception of AXOS which targets the distal colon and the rectum.

Polysaccharides such as beta-glucan increase numbers of *Bacteroides* species and *Clostridium beijerinckii*, but doesn't affect numbers of *Bifidobacterium* or *Lactobacillus* species. Guar gum is another polysaccharide that helps to lower pH and increases numbers of the beneficial *Streptococcus thermophilus.* These polysaccharides have the greatest effect on the microbial populations in the ascending and transverse colon.

Resistant starch (RS) such as RS-4 increases numbers of *Bifidobacterium* and *Parabacteroides distasonis* while decreasing numbers of *Firmicutes.* RS-4 and its analogs increase numbers of butyrate-producing *Ruminococcus bromii.* The present invention uses a resistant fiber RS-4, that has been chemically cross-linked by a sulfur linkage in order to make it resistant to normal digestive enzymes throughout the first two segments of the colon, namely the ascending and transverse sections. In this chemically cross-linked form, RS-4 makes it intact to the descending colon and rectum where it can be digested by bacteria in an environment with a pH of 6.8 to 7.0

Resistant starch has its greatest impact on microbial communities in the transverse and descending colon as well as some activity in the rectum.

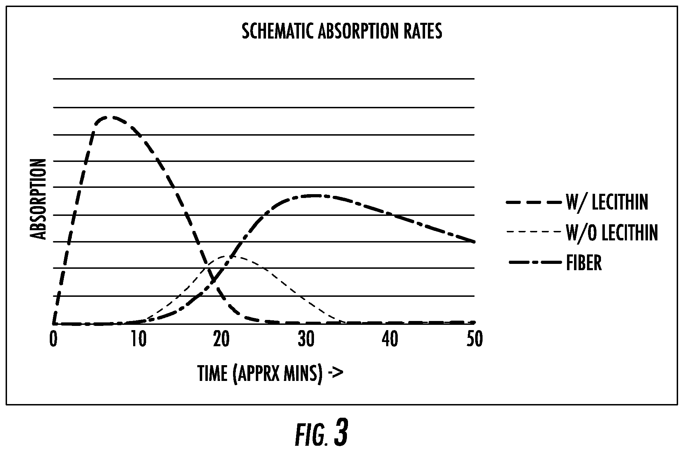

Another direct advantage of the instant invention is ability to allow L-glutamine and other polar mucogenic amino acid(s) to provide their beneficial gut lining healing and reinforcement prior to increased gut activity due to the aforementioned prebiotics. As mentioned above, this advantage is achieved primarily by the inclusion of a polar lipid, in this case lecithin, which advantageously increases the bioavailability of the aforementioned amino acids. FIG. 3 illustrates a plot of absorption rate of amino acids vs. time in minutes. As may be seen in this view, absorption with lecithin present is significantly earlier than without lecithin, and significantly earlier than with fiber alone.

One or more additional constituents may be included. One such preferred additional constituent consists of mannan or mannan oligosaccharides (MOS), which are saccharides that bind pathogens and cause them to be excreted. Mannan and mannan oligosaccharides are similar to receptors found on the surface of enterocytes and colonocytes that are targeted by pathogens. Mannan and mannan oligosaccharides in the dietary supplement bind tightly to the pathogens and prevent them from attaching to the gut lining, thereby causing them to be excreted. In the dietary supplement of the present invention, the mannan and mannan oligosaccharides are naturally derived from the cell wall of saccharomyces cerevisiae (brewer's yeast), a yeast extract, although other sources of mannan oligosaccharides are also acceptable.

In order to keep the various constituents of the dietary supplement from separating, an emulsifier may also be used. One such emulsifier is guar gum (also known as guaran), a galactomannan oligosaccharide which is extracted from the seed of the leguminous shrub Cyamopsis tetragonoloba. Guar gum is commonly used as an emulsifier, a thickener and a stabilizer. It also acts as a prebiotic fiber.

In an embodiment, additional ingredients may be included in the dietary supplement of the present invention to bind and eliminate pathogenic bacteria, to absorb and sequester pathogens, to absorb or soak up mycotoxins, and to support the renewal and growth of the cells lining the gut.

Optionally, a nutricine may be used that binds to pathogens and passes through the digestive system together with the bound pathogen and is excreted in the feces. This additional constituent consists of mannan or mannan oligosaccharides (MOS), which are complex sugars that are used to bind pathogens and, at the same time, nourish beneficial bacteria. Mannan or mannan oligosaccharides bind to attachment sites on pathogenic bacteria, preventing the pathogenic bacteria from binding to receptors in the enterocyte membrane. The mannan or mannan oligosaccharides are naturally derived from the cell wall of saccharomyces cerevisiae (brewer's yeast), a yeast extract, although other sources of mannan or mannan oligosaccharides are also acceptable.

Optionally, a pathogenic bacteria absorbent material may be used that attracts bacteria and passes through the digestive system together with the absorbed pathogenic bacteria is a pathogen absorbant such as the material marketed under the trademark SAFMANNAN by S.I. Lesaffre, Cedex, France. Other pathogenic bacteria absorbent nutricines that could instead be used include the material marketed under the trademark BIOSAF by S.I. Lesaffre, the material marketed under the trademark BIO-MOS by Alltech, Inc., in Nicholasville, Ky., as well as any other mannan oligosaccharide (complex mannose sugars derived from the cell wall of yeast).

Optionally, a mycotoxin absorbent also based upon saccharomyces cerevisiae may also be used to absorb or soak up mycotoxins in the colon. One such mycotoxin absorbent nutricine is a material marketed under the registered trademark MYCOSORB by Alltech, Inc. Other mycotoxin absorbent nutricines that could instead be used include the material marketed under the trademark MYCOFIX PLUS by Biomin Distribution, Inc. and the material marketed under the trademark D-MYCOTOC by Kanzy Medipharm, Inc.

An optional active ingredient which may be included in the dietary supplement of the present invention consists of a supplement which contains nucleotides, which can be incorporated into the rapidly renewing gut lining. Dividing cells can use exogenous nucleotides to enhance their replication. The gut wall has a number of minute finger-shaped processes of the mucous membrane called villi that serve in the absorption of nutriments, with crypts located between adjacent villi. The crypts host stem cells that proliferate and push enterocytes up the length of the villi, continuously renewing the tissues. Studies have demonstrated that dietary nucleotides increase villi height, which in turn increases the uptake of nutrients into the body and the effectiveness of other nutritional elements. There are several sources for nucleotides, the best of which are derived from brewer's or baker's yeast.

Finally, there is a non-active ingredient which is added to the dietary supplement of the present invention as an emulsifier in order to prevent its constituents from separating. The emulsifier used in the dietary supplement in this particular embodiment is guar gum, which also has thickening and stabilizing properties. Other emulsifiers having appropriate properties could be used instead of the guar gum, such as carrageenen, xanthan and agar.

Those skilled in the art will immediately appreciate that the dietary supplement of the present invention is much more than merely the sum of its ingredients, with the combination of ingredients yielding a synergistic and highly efficacious result. For example, the polar lipid acts as a spreading agent that enhances the efficacy and speed of action of the polar amino acids by enhancing their transport across the cellular membranes lining the entire digestive tract. The prebiotic fiber slows down the passage of the polar lipid and the amino acids, giving them both more time to provide their beneficial effects on the digestive tract. The amino acids also increase the integrity of the gut membrane by increasing the numbers of tight junctions, but are much more effective and show faster action in combination with the polar lipid than they would be without it.

The relative ranges of amounts of each of the ingredients, and their preferred amounts, are hereby discussed.

Amino acids: The nutricines that increase the integrity of the mucous gut membrane in this embodiment of the present invention include L-glutamine and at least one of the mucogenic amino acids mentioned above.

The range of amounts of the at least one mucogenic amino acid is between approximately 0% and approximately 10% each of the dietary supplement by weight. As one non-limiting example using L-threonine, L-threonine is approximately 10% of the dietary supplement by weight.

The range of amounts of L-glutamine is between approximately 1% and approximately 20% of the dietary supplement by weight. However, it is believed that less than 2% percent of L-glutamine will result in a reduced efficacious result. The as a non-limiting example, the amount of L-glutamine is approximately 5% of the dietary supplement by weight.

Polar lipids: The concentration of polar lipids in the present invention may vary from approximately 1% to approximately 15% of the dietary supplement by weight. Sunflower or another vegetable oil may be added as a thinner to produce a liquid dietary supplement. The polar lipids, typically in the form of lecithin, are derived from oils such as oat oil, sunflower oil, safflower oil, corn oil or soy oil. These polar lipids may be thinned out with other vegetable oils to create liquid or paste formulations of the present invention.

Prebiotics: The range of amounts of prebiotics discussed above, i.e. FOS, beta-glucan, AXOS, and RS-4 is between approximately 1% and approximately 40% each of the dietary supplement by weight. The preferred amount of prebiotic fiber is between approximately 1% and approximately 40% of the dietary supplement by weight. The most preferred amount of prebiotic fiber is approximately 25%of the dietary supplement by weight. FOS may be derived, for example, from yacon root, chicory root, Jerusalem artichoke, blue agave, acacia, or other fiber rich vegetable. Beta-glucan may, for example, be derived from oats, barley, mushrooms, seaweed, algae, or yeast cell walls. AXOS may, for example, be derived from the bran tissues of wheat, oats, barley, rice, millet, psyllium, flax, or rye. RS-4 may be derived from oats, yacon root, chicory root, flax, acacia, corn, or bacterial fermentation, and then subjected to chemical cross-linking in order to decrease digestibility by human acids and enzymes.

In those embodiments including a nutricine that binds to and eliminates pathogenic bacteria in the digestive tract, such as for example pure mannan or mannan oligosaccharide, the same may be present in approximately 0.5% to approximately 20% of the dietary supplement by weight.

In those embodiments also including an emulsifier for preventing the constituents of the dietary supplement from separating such as for example guar gum, the same may be present in approximately 1% to approximately 5% of the dietary supplement by weight.

The dietary supplement of the present invention can be manufactured as a solid, as a granulated solid, as a powder, as a paste, or as a liquid. In order to manufacture it as a solid, a small amount of oat bran or oat flour (or substitutes therefor) are added to thicken it to food bar form. It may also be pressed into a pill form. It may be added to additional ingredients to make a standard size health bar. By adding a higher percentage of oat bran or oat flour while stirring the mixture, a granular form of the supplement may be manufactured. This granular form can be sprinkled on cereal or fruit, or added to a liquid. By adding still more flour while stirring the mixture, a powder form of the supplement may be manufactured.

By adding more oil (oat oil, sunflower oil, safflower oil, or another oil), the mixture can be brought to a paste having the consistency of peanut butter. In the paste form, the dietary supplement of the present invention may be stored in gelatin capsules (as liquid-filled softgel capsules), which also provide for a consistent dosage of the dietary supplement. By adding still more oil, it can be made into a viscous liquid which can be taken by spoon.

It is desirable that the dietary supplement of the present invention is taken on a regular basis, which in the preferred embodiment is daily in order to maintain an optimal level of the ingredients in the digestive tract. The preferred dosage is between approximately one-half teaspoon and approximately three tablespoons daily. The dietary supplement of the present invention can be taken orally at least once, and possibly twice or three times, daily.

The weight of the dietary supplement varies according to its form, with the paste form having a specific density of approximately 0.8, and the granular or flour forms having a specific density of between 0.5 and 0.6. Thus, the preferred dosage of the dietary supplement of the present invention may vary between approximately one gram and approximately thirty grams per day.

Since the dietary supplement of the present invention increases the absorption of nutrients (through the action of the polar lipids) into the enterocytes and colonocytes lining the gut and slows the motility of foodstuffs through the digestive tract, it will be appreciated by those skilled in the art that by orally administering a medication in conjunction with the administration of the dietary supplement, the medication will also spend more time in the digestive tract. This will increase the absorption of the medication, and thereby act to enhance the therapeutic effect of the medication. If desired, the medication can be administered at the same time the dietary supplement is administered, or mixed or suspended in the dietary supplement prior to administration of the dietary supplement.

Upon disclosure of the dietary supplement of the present invention to those skilled in the art, they will immediately appreciate that the dietary supplement is more than merely the sum of its ingredients. The combination of ingredients described yields a synergistic result substantially more efficacious than a sum of the results which would be produced if each ingredient by itself was used. In addition to the utility of the supplement of the present invention in treating and preventing various digestive tract disorders, the dietary supplement of the present invention also has utility in treating and preventing a number of immune-related disorders as well. Depending upon the particular desired application of the dietary supplement of the present invention, additional constituents such as vitamins and minerals may also be added thereto.

Examples of vitamins which could be added include vitamins B6, B12, Biotin, C, D, E, and Niacin. Examples of mineral micronutritional additives which may be added include calcium, chromium, copper, magnesium, manganese, phosphorus, potassium, selenium, vanadium, and zinc. Other amino acids such as alpha-lipoic acid (ALA) and taurine may also be added. Other supplements could be added, such as, for the example of a supplement targeted at diabetes, coenzyme Q10 (CoQ10), inositol, and evening primrose oil. Those skilled in the art will appreciate that custom formulas could be made for application with specific digestive system and immune-related disorders.

### TREATMENT TARGETS

The present invention, in one or more of its preferred embodiments, aims to prevent, ameliorate or cure diseases related to gut dysbiosis and immune issues. Inflammation brought on by a dysbiotic microbiota is at the root of dozens of chronic diseases, which are hereby organized into six categories: gut inflammation, systemic inflammation, neurological inflammation, antibiotic-induced inflammation, auto-immunity, and chemotherapy. The teachings herein contemplate not only the supplent itself, but also a method of treatment using the supplement. Such a method includes preparing an appropriately sized dose of the supplement, and administering the supplement. The method can also include first identifying a treatment population based on ailment, and/or based on concurrent treatment. Further, the method can include specifically diagnosing an individual or group with any of the digestive system or immune related disorders described herein.

### GUT INFLAMMATION

Gut inflammation is the first and most direct effect of dysbiosis. It takes the form of ulcers, IBS, IBD (including ulcerative colitis and Crohn's disease), diverticulitis, dietary insufficiencies, colon cancer, and rectal cancer. Each of these syndromes is associated with a leaky gut. The present invention includes prebiotics designed to feed commensal bacteria that in turn produce butyrate, a short-chain fatty acid. Butyrate is the principal source of metabolic energy for the enterocytes and colonocytes lining the gut. It helps to seal the tight junctions between cells, minimizing permeability and reducing the chances of inflammation. This helps to prevent and treat ulcers, IBS and IBD.

Ulcers: The polar lipid lecithin is a component of mucus, and it forms a continuous sheet-like hydrophobic layer protecting the underlying mucus and intestinal wall, providing protection from acids, peptides, and pathogens throughout the intestines. Polar lipids thus enhance the impermeability of the enterocytes lining the gut and protect against ulcers. Glutamine and threonine are known to be limiting reactants in the creation of mucus, the mucus layer is diminished in cases of ulcerative colitis (UC), making the gut susceptible to infection and disease. Oral administration of lecithin has been shown to enhance the mucus layer. In a placebo-controlled test, 53% of treated UC patients went into remission, compared with 10% of patients receiving placebo.

IBS: IBS is a disease of fluctuating gut permeability. Prebiotic fiber, polar lipids and certain amino acids can help to increase the impermeability of the gut, reducing symptoms and leading to remission over time. The caveat with IBS is to avoid treatment during a flare-up of symptoms, as the gut may be too leaky at that time to accommodate any fermentive substrates.

Diverticulitis: This application of the dietary supplement of the present invention is based on the observation that intestinal flow is improved by the presence of polar lipids and prebiotic fiber in the diet. Diverticulitis is caused by the entrapment of food particles in small intestinal pockets or diverticula. Soluble beta-glucan fiber helps to slow transit time, helping the body to better digest food. The formula contains polar lipids that help to coat the digestive system, improving its impermeability and making it more slippery. This helps to keep particles from snagging and collecting in the diverticula.

Dietary Insufficiencies in the Elderly: This application of the dietary supplement of the present invention can help the elderly deal more effectively with a compromised digestive system. This application is based upon evidence that glutamine and prebiotic fiber can help to increase intestinal muscle tone and stimulate the immune system. Soluble beta-glucan fiber is known to slow the transit of digesta, which moderates the food bolus, allowing water to be resorbed and avoiding diarrhea while at the same time providing a bulking agent that minimizes constipation. The result is better tone, more predictable elimination and less gastric distress.

Colonic/rectal Cancer: Butyrate regulates colonocyte apoptosis and differentiation, removing and replacing dysfunctional cells, thus helping to protect against colonic cancers. Studies have shown that consumption of dietary fiber is inversely correlated to large bowel cancer. Prebiotic fiber cuts the relative risk between the lowest and highest quintiles by 60%. In populations with low fiber intake, such as most of the modern world, doubling the intake of fiber can reduce the risk of colorectal cancer by 40%. In particular, fiber from cereals, including beta-glucan and FOS, has been shown to lower the risk of rectal cancer. Similar results were also found in a study with 500 Chinese subjects, expanding upon the results from Western diets.

### SYSTEMIC INFLAMMATION

Systemic inflammation gives rise to heart disease, type 2 diabetes, various cancers, allergies, and other chronic diseases. By reducing dysbiosis and lowering systemic inflammation, the present invention prevents, treats and may even cure these diseases of inflamed organs. In addition, omega-3 polyunsaturated fatty acids (n-3 PUFA), are known to help the targets of inflammation, such as the heart and pancreas, but modern diets don't include sufficient dietary omega-3s. The polar lipids in the present invention enhance the bioavailability of dietary omega-3. They have a synergistic effect on increasing plasma and RBC n-3 levels of docosahexaenoic acid (DHA) and eicosapentaenoic acid (EPA). The choline head of the lecithin polar lipid molecule attracts and binds hydrophilic substances such as oligosaccharides and thereby increases their bioavailability.

Type 2 diabetes: A dysbiotic microbiota is associated with type 2 diabetes and its comorbidities, including diabetic retinopathy, hypertension, diabetic foot ulcers and others. By buttressing the impermeability of the gut lining and limiting inflammation via the microbiota, the prebiotic fiber in the present invention can prevent, treat or cause remission in type 2 diabetes. Prebiotic fiber slows the transit of digesta, which effectively lowers the glycemic index of the meal. Soluble beta-glucan fiber is a dietary fiber that absorbs and sequesters starches and sugars, releasing them over a longer time. A low glycemic index is the result, providing a slow release of sugars to the blood. This reduces the need for insulin to respond to large swings in blood sugar levels, allowing people with a challenged pancreas to deal better with their nutrition. People with only a slight diabetic tendency may be able to forgo daily injections of insulin as long as they continue to use the dietary supplement of the present invention.

Other chronic diseases: Similarly, other diseases that are associated with inflammation can be prevented or treated by the prebiotics and amino acids, enhanced by polar lipids, in the present invention. This components of the formula act as food for the gut and food for the microbiota, both of which contribute to a tough, impermeable gut lining, greatly reducing systemic inflammation.

### NEUROLOGICAL INFLAMMATION

Neurological inflammation may evolve from the gut, via various gut-brain pathways, including the vagus nerve, hormones, and cytokines of the immune system.

Parkinson's and Alzheimer's: Parkinson's begins with protein accumulations called Lewy bodies in infected gut cells that make their way to the brain over a period of years. Alzheimer's may also represent the displacement of misfolded amyloid proteins from the gut to the brain. Depression and anxiety are also linked to a dysbiotic microbiota. By balancing the gut microbes via a mix of prebiotic fibers, Alzheimer's and Parkinson's may be prevented.

Depression and anxiety: Prebiotic fiber has also been shown to reduce depression and anxiety in both animals and humans. The mechanism here is based on the fermentation of prebiotic fiber into butyrate, which can pass the blood-brain barrier to enter the brain. Butyrate alters gene expression in the brain, nourishing and improving the health of neurons. In addition, microbes fed on selected prebiotics such as those in the present invention, can directly produce neurotransmitters including dopamine and serotonin, the targets of many antidepressants and anxiolytics.

### ANTIBIOTIC-INDUCED INFLAMMATION

Antibiotics are commonly used in hospital environments both pre- and post-operatively to reduce the chance of infection. However, oral antibiotics carry a significant risk of dysbiosis because broad-brush antibiotics will kill beneficial as well as pathogenic bacteria. Since a balanced set of bacteria is necessary to avoid the dominance of any single species, dysbiosis can lead to a toxic overload of spore-generating bacteria that can survive antibiotics. In particular, the sporulating species *Clostridium dificile* can easily take over the gut microbiota and cause illness and even death by damaging the gut lining and allowing systemic inflammation through the translocation of bacteria across the gut lining. The amino acids L-glutamine and L-threonine can help to heal and protect the gut lining, while specifically-targeted prebiotics can help to nourish a better balanced microbiota, overcoming the complications of antibiotic-induced inflammation.

Some doctors now recommend probiotic supplements, especially formulations with multiple species such as yogurt and kefir, as an ameliorative and restorative along with antibiotic treatments in order to repopulate the gut microbiota. Due to the amino acids in the current invention, the gut lining can be repaired in advance of these population growths, helping to ensure that any bacterial blooms can be contained in the gut. The locally acting prebiotics included in the current invention encourage the complex communities of bacterial species required to form a balanced microbiota. The present invention can therefore be used as an important adjunct to oral antibiotic therapies.

### AUTO-IMMUNITY

Auto-immunity may be caused by mimicry, where bacteria or their products mimic existing body tissues. When the immune system attacks these foreign particles, it can also attack normal tissues that share the antigenic properties of the pathogen. Autoimmune diseases include arthritis, lupus, type 1 diabetes, and multiple sclerosis (MS). To the extent that prebiotics reduce pathogen load and thereby mute immune response, the present invention acts to prevent the diseases of autoimmunity.

Arthritis, lupus, type 1 diabetes and MS: These autoimmune diseases are associated with dysbiosis of the microbiota. *Prevotella* bacteria are involved with the pathogenesis of rheumatoid arthritis, and prebiotics contribute to bacteria that compete with these pathogenic species. Certain strains of *Lactobacillus* are depleted in lupus leading to gut permeability, and prebiotics can rebalance the microbiota to lessen the symptoms. MS is an inflammatory disease that has a unique microbiota, with higher abundances of *Methanobrevibacter* and *Akkermansia* species. Type 1 diabetes is associated with a microbiota that is dominated by Bacteroidetes species and depleted in butyrate-producing bacteria. The prebiotics in the present invention, along with amino acids and enhanced by polar lipids, can help to prevent these diseases. To the extent that increased gut integrity improves symptoms in these diseases, the present invention can bring relief. However, once the immune system has targeted self-tissue, it is difficult or impossible to reverse it with the current state of the art.

### CHEMOTHERAPY

Chemotherapy and radiation treatments work by attacking fast-dividing cells, including those of the gut lining. Such cancer treatments can lead to leaky gut, inflammation, and thus all the diseases listed above. Specific diseases directly attributable to cancer treatments include mucositis, stomatitis, and cachexia. In addition to these side-effects of traditional cancer therapy, there are new therapies that depend on the microbiota to be effective. Because these diseases are all gut-related, they can be ameliorated by the polar lipids, prebiotic fiber and amino acids of the present invention.

Mucositis and stomatitis: Chemotherapy depletes glutamine, and this formula helps to redress that imbalance. Glutamine taken orally can significantly reduce the duration and severity of mucositis during and after radiation therapy. It has also been shown that glutamine can reduce the effects of mucositis during bone-marrow transplantation. The embodiment of the dietary supplement of the present invention for this application may include a higher percentage of glutamine-up to twenty percent (five grams per dose). As well as glutamine, the formula includes threonine, which is essential to the production of mucus. Polar lipids such as lecithin are known to increase the bioavailability of the amino acids in the formula many-fold, thus lowering the total amount of amino acids required. This is an important aspect of the dietary supplement of the present invention, since the patient may find eating or drinking to be difficult. In addition, this application of the dietary supplement of the present invention incorporates small doses of zinc and vitamin B-12, which are also known to help relieve the symptoms of mucositis and stomatitis. Thus, the dietary supplement of the present invention can help people recover faster from cancer therapies, and possibly increase the recovery rate.

Cachexia: This application of the dietary supplement of the present invention can help a person with wasting disease, or cachexia, to put on weight and thus speed their recovery. It has been established that glutamine is helpful for HIV and cancer patients who are cachexic. Glutamine is an abundant amino acid, but in times of stress, the digestive system may not get enough of it to properly maintain its high growth rate. The dietary supplement of the present invention contains glutamine along with polar lipids to improve the bioavailability of this polar amino acid. It also includes threonine, which is an integral part of the mucus-generating pathway. Mucus, in turn, helps to maintain the barrier between the body and the digesta. Enhancing this barrier may help to prevent the loss of blood or sera that can contribute to wasting.

Checkpoint inhibitors: New cancer treatments that involve immune checkpoint inhibitors depend on a well-balanced microbiota. These new therapies involve harvesting T-cells, modifying them to attack cancer cells and then re-injecting them into the patient to treat cancers such as advanced melanoma, renal-cell carcinoma and non-small cell lung cancer. Certain bacteria, especially *Clostridiales* species and *Akkermansia muciniphila*, reduce the effectiveness of immune checkpoint inhibitors. By supplying the gut with appropriate fiber, beneficial species can modulate the numbers of these detremental bactera and improve the efficacy of these particular cancer drugs. In this way, the prebiotic fibers in the present invention can augment these immune-cell therapies.

It may therefore be appreciated from the above detailed description of the preferred embodiment of the present invention that it teaches a dietary supplement which efficaciously treats digestive tract disorders, decreasing permeability by nourishing both the cells lining the gut and the beneficial bacteria of the microbiota. As a consequence, systemic inflammation is reduced or eliminated, leading to the prevention, treatment or possible cure of many chronic diseases in humans and potentially other animals as well.

The dietary supplement of the present invention consists entirely of safe and natural ingredients rather than drugs. The dietary supplement of the present invention is orally administrable, thereby making its dispensation a simple matter. The dietary supplement of the present invention may be compounded either in a paste, solid, liquid, powder or a form which may be added to liquids for delivery. The dietary supplement of the present invention can also be packaged in a manner which makes it both easy to ship and to store.

The dietary supplement of the present invention is stable and has a long shelf life, and requires no special care to be provided by the user throughout its shelf life prior to usage. The dietary supplement of the present invention is also inexpensive relative to previously known digestive tract disorder treatments and immune-related disorder treatments, thereby enhancing its market appeal and affording it the broadest possible market. Finally, all of the aforesaid advantages and objectives of the dietary supplement of the present invention and its method of administration are achieved without incurring any substantial relative disadvantage.

Although the foregoing description of the present invention has been shown and described with reference to particular embodiments and applications thereof, it has been presented for purposes of illustration and description and is not intended to be exhaustive or to limit the invention to the particular embodiments and applications disclosed. The particular embodiments and applications were chosen and described to provide the best illustration of the principles of the invention and its practical application to thereby enable one of ordinary skill in the art to utilize the invention in various embodiments and with various modifications as are suited to the particular use contemplated. All such changes, modifications, variations, and alterations should therefore be seen as being within the scope of the present invention as determined by the appended claims when interpreted in accordance with the breadth to which they are fairly, legally, and equitably entitled.

The use of the terms "a" and "an" and "the" and similar referents in the context of describing the invention (especially in the context of the following claims) is to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein.

## Claims

1. A dietary supplement for the treatment and prevention of digestive system and immune-related disorders, said digestive and immune supplement comprising:
L-glutamine for supporting the lining of a GI tract;
at least one of the following mucogenic amino acids: L-Threonine, L-Serine, L-Proline, L-Cysteine for increasing a number and productivity of goblet cells lining the GI tract;
lecithin for increasing the bioavailability and rate of absorption of L-glutamine and the at least one mucogenic amino acid;
fructo-oligosaccharide providing at least one beneficial effect on health;
beta-glucan providing at least one beneficial effect on health;
RS-4 starch providing at least one beneficial effect on health; and
arabinoxylan oligosaccharide providing at least one beneficial effect on health.

2. The dietary supplement as defined in claim 1, wherein said L-glutamine is produced by vegan bacterial fermentation of sugar beets, isolated, purified, and micronized for faster absorption; and wherein said L-glutamine comprises between approximately one percent and twenty percent of said dietary supplement by weight.

3. A dietary supplement as defined in any preceding claim, wherein said at least one mucogenic amino acid is produced by vegan bacterial fermentation, isolated and purified.

4. The dietary supplement as defined in any preceding claim, wherein said lecithin is derived from soy oil, oat oil, sunflower oil, safflower oil, or corn oil; and said lecithin comprises between approximately five percent and approximately fifteen percent of said dietary supplement by weight.

5. The dietary supplement as defined in any preceding claim, wherein said fructo-oligosaccharides are derived from yacon root, chicory root, Jerusalem artichoke, or blue agave; and wherein said fructo-oligosaccharides comprise between approximately one percent and approximately forty percent of said dietary supplement by weight.

6. The dietary supplement as defined in any preceding claim, wherein said beta-glucan is derived from oats, barley, mushrooms, seaweed, algae, or yeast cell walls; and wherein said beta-glucan comprises between approximately one percent and approximately forty percent of said dietary supplement by weight.

7. The dietary supplement as defined in any preceding claim, wherein said arabinoxylan oligosaccharide are derived from the bran tissues of wheat, oats, barley, rice, millet, psyllium, flax, or rye; and wherein said arabinoxylan oligosaccharide comprises between approximately one percent and approximately forty percent of said dietary supplement by weight.

8. The dietary supplement as defined in any preceding claim, wherein said RS-4 starch is derived from oats, yacon root, chicory root, flax, acacia, corn or bacterial fermentation and then subjected to chemical cross-linking in order to decrease digestibility by human acids and enzymes; and wherein said RS-4 starch comprises between approximately one percent and forty percent of said dietary supplement by weight.

9. The dietary supplement as defined in any preceding claim, additionally comprising a nutricine that binds to and eliminates pathogenic bacteria in the digestive tract, the nutricine including at least one of pure mannan or mannan oligosaccharide (MOS); and wherein said nutricine that binds to and eliminates pathogenic bacteria in the digestive tract comprises between approximately one-half percent and approximately forty percent of said dietary supplement by weight.

10. The dietary supplement as defined in any preceding claim, additionally comprising guar gum in an amount of approximately one to five percent of said dietary supplement by weight.

11. The dietary supplement as defined in any preceding claim, wherein said dietary supplement is compounded as at least one of a solid food bar or pressed into a pill form, as a paste, as a granulated solid, as a powder, as a liquid or as liquid-filled soft gel capsules,

12. The dietary supplement as defined in any preceding claim, wherein said digestive system disorders are selected from a group comprising ulcers, colitis, irritable bowel syndrome, diverticulosis, diverticulitis, antibiotic-induced inflammation, Crohn's disease, mucositis, and stomatitis; or wherein said digestive system related disorders are selected from a group comprising cachexia, lactose intolerance, and dietary insufficiencies in the elderly.

13. The dietary supplement as defined in any preceding claim, wherein said immune system related disorders are selected from a group comprising arthritis, diabetes, depression, anxiety, and heart disease; or wherein said immune system related disorders are selected from a group comprising Alzheimer's, Parkinson's, multiple sclerosis, and other neurodegenerative diseases.

14. The dietary supplement as defined in any preceding claim, additionally comprising at least one vitamin from the group consisting of vitamin B6, vitamin B12, Biotin, vitamin C, vitamin D, vitamin E, and Niacin.

15. The dietary supplement as defined in any preceding claim, additionally comprising at least one mineral micronutritional additive from the group consisting of calcium, chromium, copper, manganese, magnesium, phosphorus, potassium, selenium, vanadium, and zinc.

16. A dietary supplement as defined in any one of the preceding claims for use in treating and preventing dysbiosis and diseases associated with dysbiosis.

## Patentansprüche

1. Nahrungsergänzungsmittel zur Behandlung und Vorbeugung von Erkrankungen des Verdauungssystems und des Immunsystems, wobei das Verdauungs- und Immunsystemergänzungsmittel umfasst:
L-Glutamin zur Unterstützung der Auskleidung des GI-Trakts;
mindestens eine der folgenden schleimbildenden Aminosäuren: L-Threonin, L-Serin, L-Prolin, L-Cystein zur Erhöhung der Anzahl und Produktivität der Becherzellen, die den GI-Trakt auskleiden;
Lecithin zur Erhöhung der Bioverfügbarkeit und der Absorptionsgeschwindigkeit von L-Glutamin und der mindestens einen schleimbildenden Aminosäure;
Fructo-Oligosaccharid, das mindestens eine gesundheitsfördernde Wirkung bereitstellt;
Beta-Glucan, das mindestens eine gesundheitsfördernde Wirkung bereitstellt;
RS-4-Stärke, die mindestens eine gesundheitsfördernde Wirkung bereitstellt;
Arabmoxylan-Oligosaccharid, das mindestens eine gesundheitsfördernde Wirkung bereitstellt.

2. Nahrungsergänzungsmittel nach Anspruch 1, wobei das L-Glutamin durch vegane bakterielle Fermentation von Zuckerrüben hergestellt, isoliert, gereinigt und zur schnelleren Absorption mikronisiert wird; und wobei das L-Glutamin zwischen ungefähr ein Prozent und zwanzig Prozent des Gewichts des Nahrungsergänzungsmittels umfasst.

3. Nahrungsergänzungsmittel nach einem der vorhergehenden Ansprüche, wobei die mindestens eine schleimbildende Aminosäure durch vegane bakterielle Fermentation hergestellt, isoliert und gereinigt wird.

4. Nahrungsergänzungsmittel nach einem der vorhergehenden Ansprüche, wobei das Lecithin aus Sojaöl, Haferöl, Sonnenblumenöl, Distelöl oder Maisöl gewonnen wird; und wobei das Lecithin zwischen ungefähr fünf Prozent und ungefähr fünfzehn Prozent des Gewichts des Nahrungsergänzungsmittels umfasst.

5. Nahrungsergänzungsmittel, nach einem der vorhergehenden Ansprüche, wobei die Fructo-Oligosaccharide aus Yaconwurzel, Zichorienwurzel, Topinambur oder blauer Agave gewonnen werden; und wobei die Fructo-Oligosaccharide zwischen ungefähr ein Prozent und ungefähr vierzig Prozent des Gewichts des Nahrungsergänzungsmittels umfassen.

6. Nahrungsergänzungsmittel, nach einem der vorhergehenden Ansprüche, wobei das Beta-Glucan aus Hafer, Gerste, Pilzen, Algen oder Hefezellwänden gewonnen wird; und wobei das Beta-Glucan zwischen ungefähr ein Prozent und ungefähr vierzig Prozent des Gewichts des Nahrungsergänzungsmittels umfasst.

7. Nahrungsergänzungsmittel nach einem der vorhergehenden Ansprüche, wobei das Arabinoxylan-Oligosaccharid aus den Kleiegeweben von Weizen, Hafer, Gerste, Reis, Hirse, Psyllium, Flachs oder Roggen gewonnen wird; und wobei das Arabinoxylan-Oligosaccharid zwischen ungefähr ein Prozent und ungefähr vierzig Prozent des Gewichts des Nahrungsergänzungsmittels umfasst.

8. Nahrungsergänzungsmittel nach einem der vorhergehenden Ansprüche, wobei die RS-4-Stärke aus Hafer, Yaconwurzel, Zichorienwurzel, Flachs, Akazie, Mais oder bakterieller Fermentation gewonnen und dann einer chemischen Vernetzung unterzogen wird, um die Verdaulichkeit durch menschliche Säuren und Enzyme zu verringern; und wobei die RS-4-Stärke zwischen ungefähr ein Prozent und vierzig Prozent des Gewichts des Nahrungsergänzungsmittels umfasst.

9. Nahrungsergänzungsmittel nach einem der vorhergehenden Ansprüche, das zusätzlich ein Nutricin umfasst, das sich an pathogene Bakterien im Verdauungstrakt bindet und diese eliminiert, wobei das Nutricin mindestens eines von reinem Mannan oder Mannan-Oligosaccharid (MOS) enthält; und wobei das Nutricin, das sich an pathogene Bakterien im Verdauungstrakt bindet und diese eliminiert, zwischen ungefähr ein halbes Prozent und ungefähr vierzig Prozent des Gewichts des Nahrungsergänzungsmittels umfasst.

10. Nahrungsergänzungsmittel nach einem der vorhergehenden Ansprüche, das zusätzlich Guaran in einer Menge von ungefähr ein bis fünf Prozent des Gewichts des Nahrungsergänzungsmittels umfasst.

11. Nahrungsergänzungsmittel nach einem der vorhergehenden Ansprüche, wobei das Nahrungsergänzungsmittel als mindestens eines von einem festen Lebensmittelriegel oder in Pillenform gepresst, als eine Paste, als ein granulierter Feststoff, als ein Pulver, als eine Flüssigkeit oder als mit Flüssigkeit gefüllte Weichgelkapseln zusammengesetzt ist.

12. Nahrungsergänzungsmittel nach einem der vorhergehenden Ansprüche, wobei die Erkrankungen des Verdauungssystems aus einer Gruppe ausgewählt sind, die Geschwüre, Colitis, Reizdarmsyndrom, Divertikulose, Divertikulitis, durch Antibiotika hervorgerufene Entzündungen, Morbus Crohn, Mukositis und Stomatitis umfasst; oder wobei die mit dem Verdauungssystem zusammenhängenden Erkrankungen aus einer Gruppe ausgewählt sind, die Kachexie, Laktoseintoleranz und diätetische Unzulänglichkeiten bei älteren Menschen umfasst.

13. Nahrungsergänzungsmittel, nach einem der vorhergehenden Ansprüche, wobei die mit dem Immunsystem zusammenhängenden Erkrankungen aus einer Gruppe ausgewählt sind, die Arthritis, Diabetes, Depression, Angstzustände und Herzerkrankungen umfasst; oder wobei die mit dem Immunsystem zusammenhängenden Erkrankungen aus einer Gruppe ausgewählt sind, die Alzheimer, Parkinson, Multiple Sklerose und andere neurodegenerative Krankheiten umfasst.

14. Nahrungsergänzungsmittel nach einem der vorhergehenden Ansprüche, das zusätzlich mindestens ein Vitamin aus der Gruppe bestehend aus Vitamin B6, Vitamin B12, Biotin, Vitamin C, Vitamin D, Vitamin E und Niacin umfasst.

15. Nahrungsergänzungsmittel nach einem der vorhergehenden Ansprüche, zusätzlich umfassend mindestens einen mineralischen Mikronährstoffzusatz aus der Gruppe bestehend aus Calcium, Chrom, Kupfer, Mangan, Magnesium, Phosphor, Kalium, Selen, Vanadium und Zink.

16. Nahrungsergänzungsmittel nach einem der vorhergehenden Ansprüche, zur Verwendung bei der Behandlung und Vorbeugung von Dysbiose und mit Dysbiose verbundenen Krankheiten.

## Revendications

1. Supplément diététique pour le traitement et la prévention de troubles du système digestif et en lien avec le système immunitaire, ledit supplément digestif et immunitaire comprenant :
de la L-glutamine pour aider à recouvrir le conduit gastro-intestinal ;
au moins l'un des acides aminés mucogènes suivants : de la L-thréonine, L-sérine, L-proline, L-cystéine, pour augmenter le nombre et la productivité des cellules caliciformes qui recouvrent le conduit gastro-intestinal ;
de la lécithine pour augmenter la biodisponibilité et le taux d'absorption de la L-glutamine et de l'au moins un acide aminé mucogène ;
un fructo-oligosaccharide fournissant au moins un effet positif sur la santé ;
un bêta-glucane fournissant au moins un effet positif sur la santé ;
de l'amidon RS-4 fournissant au moins un effet positif sur la santé ;
un oligosaccharide arabinoxylane fournissant au moins un effet positif sur la santé ;

2. Supplément diététique tel que défini à la revendication 1, dans lequel ladite L-glutamine est produite par fermentation bactérienne végane de betteraves sucrières, isolée, purifiée et micronisée pour une absorption plus rapide ; et dans lequel ladite L-glutamine comprend entre environ 1 % et 20 % en poids dudit supplément diététique.

3. Supplément diététique tel que défini dans l'une quelconque des revendications précédentes, dans lequel ledit au moins un acide aminé mucogène est produit par fermentation bactérienne végane, isolé et purifié.

4. Supplément diététique tel que défini dans l'une quelconque des revendications précédentes, dans lequel ladite lécithine est dérivée d'huile de soja, d'huile d'avoine, d'huile de tournesol, d'huile de carthame ou d'huile de maïs ; et ladite lécithine contient entre environ 5 % et environ 15 % en poids dudit supplément diététique.

5. Supplément diététique tel que défini dans l'une quelconque des revendications précédentes, dans lequel lesdits fructo-saccharides sont dérivés de racine de yacon, de racine de chicorée, de topinambour ou d'agave bleu, et dans lequel lesdits fructo-saccharides contiennent entre environ 1 % et environ 40 % en poids dudit supplément diététique.

6. Supplément diététique tel que défini dans l'une quelconque des revendications précédentes, dans lequel ledit bêta-glucane est dérivé d'avoine, d'orge, de champignon, de varech, d'algue ou de parois de cellules de levure ; et dans lequel ledit bêta-glucane contient entre environ 1 % et environ 40 % en poids dudit supplément diététique.

7. Supplément diététique tel que défini dans l'une quelconque des revendications précédentes, dans lequel ledit oligosaccharide arabinoxylane est dérivé de son de blé, d'avoine, d'orge, de riz, de millet, d'ispaghul, de lin ou de seigle ; et dans lequel ledit oligosaccharide arabinoxylane contient entre environ 1 % et environ 40 % en poids dudit supplément diététique.

8. Supplément diététique tel que défini dans l'une quelconque des revendications précédentes, dans lequel ledit amidon RS-4 est dérivé d'avoine, de racine de yacon, de racine de chicorée, de lin, d'acacia, de maïs ou de fermentation bactérienne, puis est soumis à réticulation chimique afin de réduire sa digestibilité par les acides en enzymes humains ; et dans lequel ledit amidon RS-4 contient entre environ 1 % et environ 40 % en poids dudit supplément diététique.

9. Supplément diététique tel que défini dans l'une quelconque des revendications précédentes, contenant en plus une nutricine qui se lie et élimine des bactéries pathogènes dans le conduit digestif, la nutricine comprenant au moins un entre un mannane pur ou un mannane oligosaccharide (MOS) ; et dans lequel ladite nutricine qui se lie et élimine des bactéries pathogènes dans le conduit digestif contient entre environ 1,5 % et environ 40 % en poids dudit supplément diététique.

10. Supplément diététique tel que défini dans l'une quelconque des revendications précédentes, contenant en plus de la gomme de guar à hauteur d'environ 1 à 5 % en poids dudit supplément diététique.

11. Supplément diététique tel que défini dans l'une quelconque des revendications précédentes, ledit supplément diététique étant constitué d'au moins un parmi une barre alimentaire solide ou comprimé sous la forme d'une pilule, d'une pâte, d'un solide granulé, d'une poudre, d'un liquide ou de capsules de gel souple remplies de liquide.

12. Supplément diététique tel que défini dans l'une quelconque des revendications précédentes, lesdits troubles du système digestif étant choisis dans un groupe comprenant des ulcères, une colique, le syndrome du côlon irritable, une diverticulose, une diverticulite, une inflammation induite par antibiotique, la maladie de Crohn, une mucosité et une stomatite ; ou lesdits troubles liés au système digestif étant choisis dans un groupe comprenant la cachexie, l'intolérance au lactose et les insuffisances diététiques des personnes âgées.

13. Supplément diététique tel que défini dans l'une quelconque des revendications précédentes, lesdits troubles du système immunitaire étant choisis dans un groupe comprenant l'arthrite, le diabète, la dépression, l'anxiété et la cardiopathie ; ou lesdits troubles du système immunitaire étant choisis dans un groupe comprenant la maladie d'Alzheimer, la maladie de Parkinson, la sclérose en plaques et d'autres maladies neurodégénératives.

14. Supplément diététique tel que défini dans l'une quelconque des revendications précédentes, comprenant en plus au moins une vitamine dans le groupe constitué de la vitamine B6, la vitamine B12, la biotine, la vitamine C, la vitamine D, la vitamine E et la niacine.

15. Supplément diététique tel que défini dans l'une quelconque des revendications précédentes, comprenant en plus au moins un additif micronutriment minéral dans le groupe constitué du calcium, du chrome, du cuivre, du manganèse, du magnésium, du phosphore, du potassium, du sélénium, du vanadium et du zinc.

16. Supplément diététique tel que défini dans l'une quelconque des revendications précédentes, destiné à être utilisé dans le traitement et la prévention de la dysbiose et de maladies associées à la dysbiose.
